# EUROPEAN PATENT APPLICATION

(11) **EP 1 498 139 A1**
(43) Date of publication of application: **19.01.2005**
(21) Application number: 03746171.2
(22) Date of filing: 11.04.2003
(51) Int. Cl.: A61K 45/00, A61K 31/47, A61P 7/02, A61P 31/04, A61P 43/00

(54) **NOVEL THROMBOMODULIN EXPRESSION PROMOTERS**

(30) Priority: 12.04.2002 JP 2002111247
(71) Applicant: Kowa Company Ltd., Nagoya-shi Aichi-ken, 460-8625 (JP); Nissan Chemical Industries, Ltd., Chiyoda-ku, Tokyo 101-0054 (JP)
(72) Inventor: OIDA, Koji, Yoshida-gun, Fukui 910-1104 (JP)
(74) Representative: Cresswell, Thomas Anthony
(86) International application number: PCT/JP2003/004627
(87) International publication number: WO 2003/086466

(57) **Abstract**

It is intended to provide a novel pharmaceutical effect of HMG-CoA reductase inhibitors, in particular, (+)-(3R, 5S, 6E)-7-[2-cyclopropyl-4-(4-fluorophenyl)-3-quinolyl]-3,5-di hydroxy-6-heptenoic acid or its salt. Namely, TM expression promoters, more specifically speaking, antithrombotic drugs, preventive/remedies for sepsis, antiplatelet drugs and anticoagulants comprising as the active ingredient an HMG-CoA reductase inhibitor, in particular, (+)-(3R, 5S, 6E)-7-[2-cyclopropyl-4-(4-fluorophenyl)-3-quinolyl]-3,5-dihydroxy-6 - heptenoic acid or its salt.

## Description

### Technical Field

The present invention relates to an HMG-CoA reductase inhibitor, in particular, to a thrombomodulin expression promoter comprising (+)-(3R, 5S, 6E)-7-[2- cyclopropyl-4-(4-fluorophenyl)-3-quinolyl]-3,5-dihydroxy-6-heptenoic acid or its salt (hereinafter abbreviated as pitavastatin in some cases) as an active ingredient. Thrombomodulin is an anticoagulant substance and useful as a preventive and therapeutic agent for a disease associated with coagulation disorder and/or sepsis. Also, the present invention relates to an antithrombotic agent, a preventive/ therapeutic agent for sepsis, an antiplatelet agent and an anticoagulant comprising pitavastatin or its salt as an active ingredient.

### Background Art

Thrombomodulin (hereinafter abbreviated as TM in some cases) is one of the glycoproteins, which is present on the vascular endothelial cell membrane. It binds to thrombin as a coenzyme for the activation of protein C by thrombin, and it functions mainly as a blood-clotting regulator.

In addition, it has been shown recently that activated protein C is reduced in patients with sepsis, and TM, which is a coenzyme for the activation of protein C, has been highly expected to be used as a preventive/ therapeutic agent for sepsis (N Engl J Med, Vol. 344, No. 10 759-762 (2001)).

It has been shown recently that an HMG-CoA reductase inhibitor (hereinafter abbreviated as statin in some cases) has, in addition to the original effect on lowering LDL cholesterol, an effect on other than lipid metabolism, namely a pleiotropic effect such as improvement of endothelial cell function, inhibition of smooth muscle cell proliferation and migration, stabilization of plaque (inhibition of macrophage foam cell formation, inhibition of matrix protease (MMP) expression or the like), an antithrombotic effect or an antioxidant effect.

With respect to the antithrombotic effect of statin, improvement of an antithrombotic effect on endothelial cells by inhibition of LDL oxidation (Atherosclerosis, Vol. 138, p271-280 (1998)), an inhibitory effect on activation of coagulation factor VII (Atheroscler Tromb Vase Biol, Vol. 17, p265-272 (1997)), an inhibitory effect on platelet hyperfunction (Atheroscler Tromb Vasc Biol, Vol. 15, p247-251 (1995)), an inhibitory effect on platelet adhesion (Atheroscler Tromb Vasc Biol, Vol. 19, p1812-1817 (1999)) and the like were reported in the past.

However, a promoting effect of statin on TM expression was totally unknown.

### Disclosure of the invention

The present invention provides an HMG-CoA reductase inhibitor, in particular, a novel pharmacological effect of (+)-(3R, 5S, 6E)-7-[2-cyclopropyl-4-(4-fluorophenyl)-3-quinolyl]-3,5- dihydroxy-6-heptenoic acid or its salt. More particularly, the present invention provides a medicinal agent which can regulate TM expression.

### Brief Description of the Drawings

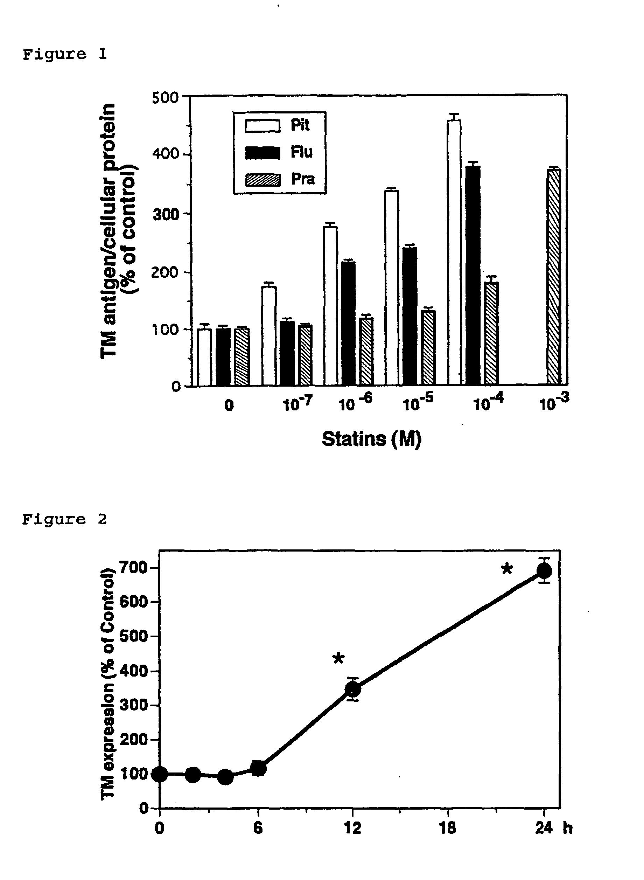

Fig. 1 shows the results of changes in the levels of thrombomodulin antigen due to the statin of the present invention. The vertical axis of Fig. 1 shows the ratio (%) of the TM antigen level/ cellular protein to the control. The horizontal axis shows the concentration of each statin. The open bar, the black bar and the shaded bar in each concentration show pitavastatin (Pit), fluvastatin (Flu) and pravastatin (Pra), respectively.
Fig. 2 shows the time course of the amounts of thrombomodulin expression due to the pitavastatin of the present invention. The vertical axis of Fig. 2 shows the ratio (%) of the TM expression level to the control. The horizontal axis shows the time (hour).

### Best Mode for Carrying Out the Invention

The present inventors explored a substance which affects the expression of TM mRNAs and proteins by using vascular endothelial cells. As a result, they totally surprisingly found that a statin has a promoting effect on TM expression, and furthermore, particularly pitavastatin has a strong promoting effect on TM expression, and thus the present invention has been completed.

Namely, the present invention provides a TM expression promoter comprising an HMG-CoA reductase inhibitor, in particular, (+)-(3R,5S,6E)-7-[2-cyclopropyl-4-(4-fluorophenyl)-3-quino lyl]-3,5-dihydroxy-6-heptenoic acid or its salt as an active ingredient.

Although a statin is a compound known as a therapeutic agent for hyperlipidemia, it is totally unknown whether a statin acts on TM expression or not.

There is no particular restriction on the statin, however, it is described in, for example, US Patent No. 4,739,073 and European Patent Application Laid-open No. 114,027; European Patent Application Laid-open No. 367,895; US Patent Nos. 5,001,255, 4,613,610, 4,851,427, 4,755,606 and 4,808,607, 4,751,235, 4,939,159, 4,822,799, 4,804,679, 4,876,280, 4,829,081, 4,927,851, 4,588,715; and F.G. Kathawala, Medical Research Reviews, 11, 121-146 (1991), in addition, European Patent Application Laid-open No. 304,063; European Patent Application Laid-open No. 330,057 and US Patent Nos. 5,026,708 and 4,868,185; European Patent Application Laid-open No. 324,347; European Patent Application Laid-open No. 300,278; US Patent Nos. 5,013,749, 5,872,130 and 5,856,336, US Patent No. 4,231,938, US Patent No. 4,444,784, US Patent No. 4,346,227, US Patent No. 5,354,772, US Patent No. 5,273,995, US Patent No. 5,177,080, US Patent No. 3,983,140, Japanese Patent No. 2,648,897, US Patent No. 5,260,440 or Bioorganic & Medicinal Chemistry, 5, pp437, (1977) and Japanese Patent No. 2,569,746, European Patent No. 304,063 or US Patent No. 5,856,336.

In particular, lovastatin is described in US Patent No. 4,231,938; simvastatin in US Patent No. 4,444,784, pravastatin in US Patent No. 4,346,227; fluvastatin in US Patent No. 5,354,772; atorvastatin in US Patent No. 5,273,995; cerivastatin in US Patent No. 5,177,080; mevastatin in US Patent No. 3,983,140; in addition, rosuvastatin, namely monocalcium bis(+)-7-[4-(4-fluorophenyl)-6-isopropyl-2-(N-methyl-N-met hansulfonylaminopyrimidine)-5-yl]-(3R,5S)-dihydroxy-(E)-6-heptenoate in Japanese Patent No. 2.648,897, US Patent No. 5, 260, 440 or Bioorganic & Medicinal Chemistry, 5, pp437, (1977). Similarly, pitavastatin calcium is described in Japanese Patent No. 2,569,746, European Patent No. 304,063 or US Patent No. 5,856,336.

The TM expression promoter of the present invention has an anticoagulant effect and an inhibitory effect on platelet aggregation, and is useful as an antithrombotic (antiplatelet agent and/or anticoagulant) and/or a preventive/ therapeutic agent for sepsis in the prevention and treatment of a disease for which an anticoagulant activity is desired to be controlled.

Examples of a dosage form in the case where the TM expression promoter of the present invention is used as a drug include an oral administration forms such as tablet, capsule, granule, powder, syrup or the like, or a parenteral administration forms such as intravenous injection, intramuscular injection, suppository, inhalant, percutaneous absorption preparation, ophthalmic solution, nasal solution or the like. To prepare medicinal preparations in various dosage forms as above, this active ingredient can be used solely or in combination with other pharmaceutically acceptable excipients, binders, fillers, disintegrants, surfactants, lubricants, disparsants, buffers, preservatives, corrigents, flavors, coatings, carriers, diluents and the like as needed. Among these dosage forms, a preferred form is the oral administration. When a preparation for oral administration is prepared, it is preferable that the pH be adjusted according to the description of, for example, JP-A-2-6406, Patent No. 2,774,037, WO 97/23200, considering the stability of the active ingredient. The dose of the drug of the present invention varies depending on the body weight, age, sex, symptoms and the like of patients, however, generally in the case of an adult, it is preferable that the dose of 0.01 to 1000 mg/ day, particularly 0.1 to 100 mg/ day once or divided into several times a day as a compound represented by the general formula (1) be orally administered or parenterally administered.

The TM expression promoter of the present invention is orally administered or parenterally administered.

The dose of the TM expression promoter of the present invention varies depending on the body weight, age, sex, symptoms and the like of patients, however, generally in the case of an adult, it is preferable that the dose of 0.01 to 1000 mg/ day, preferably 0.1 to 100 mg/ day divided into one to three times a day be administered.

Incidentally, all the content described in the specification, Japanese Patent Application No. 2002-111247, is incorporated in this description.

### Examples

Hereunder, the present invention is described in more detail with reference to Examples, however, the present invention is not limited to these Examples.

### Example 1

Normal human umbilical vein endothelial cells (HUVECs) were plated at a density of 2 x 10⁵ cells/ well in a 24-well plate with HuMedia-EG2 medium (Kurabo Inc.) to which 2% (vol/vol) fetal bovine serum, 50 µg/ml gentamicin, 50 ng/ml amphotericin B, 10 ng/ml hEGF, 5 ng/ml hFGF-B, 1 µg/ml hydrocortisone and 10 µg/ml heparin were added. Subsequently, each test substance (pitavastatin, fluvastatin and pravastatin) was added to the medium at a final concentration of 10⁻⁷ M to 10⁻³ M, and incubated for 24 hours with 5% CO₂ at 37°C. After the incubation, the cells were washed 3 times with a phosphate buffer solution (pH 7.4), and suspended in 50 mmol/L Tris-HCl (pH 7.4) that contained 0.15 mol/L NaCl, 0.5% Triton X-100 and 1 mmol/L benzamide hydrchloride, then, TM was extracted. The levels of TM antigen were measured by enzyme immunoassay using the monoclonal antibodies TMmAb 2, 11 and 20. In the case where the value of the control (containing an equivalent weight of DMSO (0.01%)) is assigned to 100%, the effects on the TM antigen levels existing in the cellular proteins due to the addition of each test substance were measured.

The results are shown in Fig. 1. The vertical axis of Fig. 1 shows the ratio (%) of the TM antigen level/ cellular protein to the control. The horizontal axis shows the concentration of each statin. The open bar, the black bar and the shaded bar in each concentration show pitavastatin (Pit), fluvastatin (Flu) and pravastatin (Pra), respectively.

From the results, it is found that any statin increases the levels of TM expression. In particular, pitavastatin (Pit) was found to show significant increase level.

### Example 2

HUVECs were plated in HuMedia-EG2 medium (Kurabo Inc.) to which 2% (vol/vol) fetal bovine serum, 50 µg/ml gentamicin, 50 ng/ml amphotericin B, 10 ng/ml hEGF, 5 ng/ml hFGF-B, 1 µg/ml hydrocortisone and 10 µg/ml heparin were added. Subsequently, pitavastatin (at a final concentration of 10⁻⁵ M) was added to the medium, and incubated for the indicated time with 5% CO₂ at 37°C. Total RNA was extracted by using TRIZOL reagent (Life Technologies, Inc.) and the levels of TM m-RNA expression were measured by the Northern blot method, which is a usual method.

The results are shown in Table 2. The vertical axis of Fig. 2 shows the ratio (%) of the TM expression level to the control. The horizontal axis shows the time (time). It is found that the expression level increased drastically after 12 hours.

### Industrial Applicability

According to the present invention, a statin promotes the expression of TM which mainly functions as a blood clotting regulator, in particular, pitavastatin can significantly promote the expression of TM compared with other statins, therefore, it is useful as an antithrombotic agent and/or a preventive/ therapeutic agent for sepsis.

## Claims

1. A thrombomodulin expression promoter comprising an HMG-CoA reductase inhibitor as an active ingredient.

2. The thrombomodulin expression promoter according to claim 1, wherein the HMG-CoA reductase inhibitor is (+)-(3R, 5S, 6E)-7-[2-cyclopropyl-4-(4-fluorophenyl)-3-quinolyl]-3,5-di hydroxy-6-heptenoic acid or its salt.

3. An antithrombotic agent comprising, as an active ingredient, (+)-(3R, 5S, 6E)-7-[2-cyclopropyl-4-(4-fluorophenyl)-3-quinolyl]-3,5-dihydroxy-6-heptenoic acid or its salt.

4. A preventive/ therapeutic agent for sepsis comprising, as an active ingredient, (+)-(3R, 5S, 6E)-7-[2-cyclopropyl-4-(4-fluorophenyl)-3-quinolyl]-3,5-dihydr oxy-6-heptenoic acid or its salt.

5. An antiplatelet agent comprising, as an active ingredient, (+)-(3R, 5S, 6E)-7-[2-cyclopropyl-4-(4-fluorophenyl)-3-quinolyl]-3,5-dihydroxy-6-heptenoic acid or its salt.

6. An anticoagulant agent comprising, as an active ingredient, (+)-(3R, 5S, 6E)-7-[2-cyclopropyl-4-(4-fluorophenyl)-3-quinolyl]-3,5-dihydroxy-6-heptenoic acid or its salt.
